# EUROPEAN PATENT APPLICATION

(11) **EP 4 410 276 A1**
(43) Date of publication of application: **07.08.2024**
(21) Application number: 22876109.4
(22) Date of filing: 26.09.2022
(51) Int. Cl.: A61K 9/127, A61K 9/14, A61K 31/7088, A61K 47/14, A61K 47/22, A61K 47/28, A61K 48/00

(54) **LIPID NANOPARTICLES USED TO TRANSPORT NUCLEIC ACIDS INTO LYMPHATIC ENDOTHELIUM CELLS**

(30) Priority: 30.09.2021 JP 2021161239
(71) Applicant: NOF Corporation, Shibuya-ku Tokyo 150-6019 (JP); National University Corporation Chiba University, Chiba-shi, Chiba 263-8522 (JP)
(72) Inventor: TANGE, Kota, Kawasaki-shi, Kanagawa 210-0865 (JP); YOSHIOKA, Hiroki, Kawasaki-shi, Kanagawa 210-0865 (JP); NAKAI, Yuta, Kawasaki-shi, Kanagawa 210-0865 (JP); AKITA, Hidetaka, Chiba-shi, Chiba 260-8675 (JP); SAKURAI, Yu, Chiba-shi, Chiba 260-8675 (JP); TANAKA, Hiroki, Chiba-shi, Chiba 260-8675 (JP); YOSHIKAWA, Keito, Chiba-shi, Chiba 260-8675 (JP)
(74) Representative: J A Kemp LLP
(86) International application number: PCT/JP2022/035637
(87) International publication number: WO 2023/054242

(57) **Abstract**

The present invention provides a lipid nanoparticle used for delivering a nucleic acid to a lymphatic endothelial cell, including an ionic lipid represented by the formula (1), cholesterol, and a dimyristoylglycerol PEG with a number average molecular weight of PEG chain of 1,000 to 3,000, wherein an amount of the dimyristoylglycerol PEG is not less than 3 mol% with respect to the total of the ionic lipid represented by the formula (1) and the cholesterol (the symbols in the formula (1) are as defined in the specification).

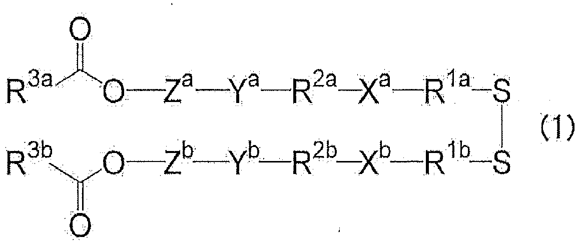

## Description

### [Technical Field]

The present invention relates to lipid nanoparticles used to deliver nucleic acids to lymphatic endothelial cells, and methods for delivering nucleic acids to lymphatic endothelial cells.

### [Background Art]

For practicalization of nucleic acid therapy, an effective and safe nucleic acid delivery carrier is demanded. While virus vectors are nucleic acid delivery carriers with good expression efficiency, they have practical problems from the aspect of safety. Therefore, the development of non-viral nucleic acid delivery carriers that can be used more safely is ongoing. Among them, lipid nanoparticles that are carriers using ionic lipids are non-viral nucleic acid delivery carriers most generally used at present.

Ionic lipids are largely constituted of amine moiety and lipid moiety. The amine moiety, which is protonated under acidic conditions, interacts electrostatically with nucleic acids, which are polyanions, to form lipid nanoparticles, which promotes uptake into cells and delivers nucleic acids into cells.

A known ionic lipid that is generally widely used is, for example, 1,2-dioleoyl-3-dimethylammonium propane (DODAP). It is known that by combining known ionic lipids with phospholipids, cholesterol, and PEG lipids, lipid nanoparticles can be formed and nucleic acids can be delivered into cells (Non Patent Literature 1).

Patent Literature 1 describes an ionic lipid having a structure in which compounds consisting of one or two amine moieties and one lipid moiety are connected by a biodegradable disulfide bond. This literature states that the ionic lipid can improve pharmacokinetics such as blood stability and tumor targeting, and that by changing the structure around the amine moiety, the pKa of a lipid membrane structure can be adjusted to a value advantageous for endosomal escape in cells, and further that it has the effect of dissociating nucleic acids from lipid membrane structures by utilizing the cleavage of disulfide bonds within cells. In fact, since it shows higher nucleic acid delivery efficiency compared to a known ionic lipid DODAP, it is clear that this ionic lipid can achieve improvement of improve the intracellular dynamics such as improvement of the delivery efficiency of nucleic acids into the cytoplasm and the like.

In Patent Literature 2, a lipid membrane structure is shown that has enhanced ability to fuse with endosomal membrane and has further improved efficiency of nucleic acid introduction into the cytoplasm, by using an ionic lipid having, in addition to a tertiary amine moiety and disulfide bond, an aromatic ring introduced near the lipid moiety.

As described above, ionic lipids with improved intracellular dynamics have been developed by increasing endosomal escape efficiency and membrane fusion ability. On the other hand, in order for lipid nanoparticles made of ionic lipids to exhibit more practical effects as nucleic acid delivery carriers in vivo, directivity to target organs and cells is demanded.

One of the PEG lipids widely used in lipid nanoparticles is dimyristoylglycerol PEG (DMG-PEG). It is known that when lipid nanoparticles using DMG-PEG are administered into the blood, the PEG lipids gradually dissociate from the lipid nanoparticles in the blood, and apolipoprotein E (ApoE) present in the blood adheres to lipid nanoparticles, thus increasing its accumulation in the liver where ApoE receptors are expressed (Non Patent Literature 2).

As an example of imparting directivity to organs other than the liver, there is an example in which accumulation in tumor was enhanced by using, as a PEG lipid, distearoylglycerol PEG (DSG-PEG) having a stearic acid-derived hydrophobic group, instead of DMG-PEG having a myristic acid-derived hydrophobic group can be mentioned (Non Patent Literature 3). Compared to DMG-PEG, DSG-PEG does not easily dissociate from lipid nanoparticles in the blood. Therefore, DSG-PEG avoids adhesion of ApoE in the blood, suppresses accumulation in the liver, and shows high retention in the blood, as a result of which increases accumulation in tumors.

As described above, there are known multiple lipid nanoparticles with improved intracellular dynamics and multiple lipid nanoparticles with controlled organ accumulation after intravenous injection by changing PEG lipid, which is one of the constituent components. However, there is a wide variety of organs and cells that can be targets for drug discovery, and the development of lipid nanoparticles having directivity to various organs and cells is demanded.

Lymphatic endothelial cells, which constitute lymphatic vessels, are known to be involved in lymph transport and immune responses, and are expected to be new drug discovery targets. However, there are no findings relating to the dynamics of lipid nanoparticles in the lymph or examples of the development of lipid nanoparticles targeting lymphatic endothelial cells.

### [Citation List]

### [Patent Literature]

[Patent Literature 1]
   US 2014/0335157 A1
[Patent Literature 2]
   WO 2019/188867 A1

### [Non Patent Literature]

[Non Patent Literature 1]
   Biomaterials, 29(24-25): 3477-3496 (2008)
[Non Patent Literature 2]
   J. Control. Release, 235: 236-244 (2016)
[Non Patent Literature 3]
   J. Control. Release, 200: 97-105 (2015)

### [Summary of Invention]

### [Technical Problem]

An object of the present invention is to provide lipid nanoparticles that can efficiently deliver nucleic acids to lymphatic endothelial cells, and to provide a method for delivering nucleic acids to lymphatic endothelial cells by using the lipid nanoparticles.

### [Solution to Problem]

The present inventors have conducted intensive studies in view of the above-mentioned problems and found that lipid nanoparticles produced using an ionic lipid that has a pKa suitable for endosomal escape and is specifically decomposed in a reducing environment within cells, and a specific ratio of dimyristoylglycerol PEG represented by the following formula (2) can efficiently deliver nucleic acids to lymphatic endothelial cells. The present invention based on this finding is as follows.
[1] A lipid nanoparticle used for delivering a nucleic acid to a lymphatic endothelial cell, comprising
   an ionic lipid represented by the formula (1): 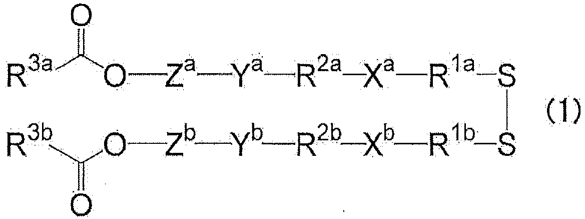
   (in the formula (1),
         R^{1a} and R^{1b} are each independently an alkylene group having 1 to 6 carbon atoms,
         X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
         R^{2a} and R^{2b} are each independently an alkylene group or an oxydialkylene group each having not more than 8 carbon atoms,
         Y^{a} and Y^{b} are each independently an ester bond, an amide bond, a carbamate bond, an ether bond or a urea bond,
         Z^{a} and Z^{b} are each independently a divalent group derived from an aromatic compound having 3 to 16 carbon atoms and at least one aromatic ring, and optionally having a hetero atom, and
         R^{3a} and R^{3b} are each independently a residue derived from a reaction product of a liposoluble vitamin having a hydroxyl group, and succinic anhydride or glutaric anhydride, or a residue derived from a reaction product of a sterol derivative having a hydroxyl group, and succinic anhydride or glutaric anhydride, or an aliphatic hydrocarbon group having 12 to 22 carbon atoms),
         cholesterol, and
         a dimyristoylglycerol PEG represented by the formula (2):

            CH₂(OR⁶)-CH(OR⁷)-CH₂(OR⁸) (2)
      (in the formula (2),
         two of R⁶, R⁷, and R⁸ are myristoyl groups, and the remaining one is an alkyl group having 1 to 6 carbon atoms connected via a polyethylene glycol chain with a number average molecular weight of 1,000 to 3,000),
         wherein an amount of the dimyristoylglycerol PEG represented by the formula (2) is not less than 3 mol% with respect to the total of the ionic lipid represented by the formula (1) and the cholesterol.
[2] The lipid nanoparticle of the aforementioned [1], wherein lipids constituting the lipid nanoparticle consist of the ionic lipid represented by the formula (1), the cholesterol, and the dimyristoylglycerol PEG represented by the formula (2).
[3] The lipid nanoparticle of the aforementioned [1] or [2], wherein the ionic lipid represented by the formula (1) is an ionic lipid represented by the following formula: 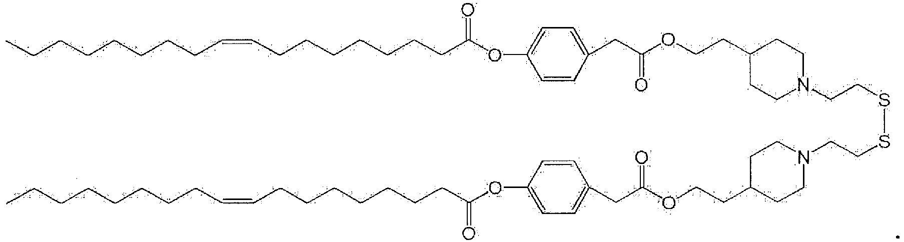
[4] A method for delivering a nucleic acid to a lymphatic endothelial cell, comprising subcutaneously administering the lipid nanoparticle of any one of the aforementioned [1] to [3] that encapsulates the nucleic acid to a subject.

### [Advantageous Effects of Invention]

The lipid nanoparticles of the present invention can efficiently deliver a nucleic acid to a lymphatic endothelial cell.

### [Brief Description of Drawings]

Fig. 1 is a diagram showing the uptake efficiency of LNPs prepared using DMG-PEG2000 (DMG) or DSG-PEG2000 (DSG) with various composition ratios, into lymphatic endothelial cells (LEC) and blood vessel endothelial cells (BEC). ** and ††: p<0.01
Fig. 2 is a diagram showing the knockdown effect of VEGFR3 gene expression in the lymphatic endothelial cells in the tail of mice subcutaneously administered with VEGFR3 siRNA-encapsulating LNP produced using DMG-PEG2000 (DMG) with different composition ratios. NT: non-administration group, siCtrl: non-specific siRNA administration group. **:p<0.01

### [Description of Embodiments]

While the embodiments of the present invention are explained in the following, the present invention is not limited thereto.

The present invention relates to lipid nanoparticles containing an ionic lipid represented by the formula (1) (i.e., ionic lipid having a tertiary amino group, a lipid moiety, and a disulfide bond as a biodegradable group), cholesterol, and dimyristoylglycerol PEG represented by the formula (2), and methods for delivering nucleic acids to lymphatic endothelial cells by using the lipid nanoparticles.

### Lipid nanoparticles

In the present specification, the "lipid nanoparticles" (Lipid Nano Particle, sometimes to be abbreviated as "LNP" in the present specification) means a particle having a membrane structure wherein the hydrophilic groups of amphiphilic lipid are arranged in the interface, facing the aqueous phase side, and having a particle size of less than 1 um, and the "amphiphilic lipid" means a lipid having both a hydrophilic group and a hydrophobic group.

The particle size of the lipid nanoparticle of the present invention is preferably 10 nm to 500 nm, more preferably 30 nm to 300 nm. The particle size can be measured by using a particle size distribution measuring device such as Zetasizer Nano (Malvern) or the like. The particle size of the lipid nanoparticles can be appropriately adjusted by the method for producing the lipid nanoparticles. In the present specification, the "particle size" means an average particle size (number average) measured by a dynamic light scattering method.

Examples of the amphiphilic lipid include ionic lipid, phospholipid, PEG lipid, and the like. In the present specification, "PEG" means polyethylene glycol, "PEG lipid" means a lipid modified with PEG, and "Y modified with X " (e.g., X:PEG, Y:lipid) means Y bound by X. In other words, "PEG lipid" means a lipid bound by PEG.

The lipid nanoparticles of the present invention may contain lipids other than the ionic lipid represented by the formula (1), cholesterol, and the dimyristoylglycerol PEG represented by the formula (2) (hereinafter sometimes to be referred to as "other lipid"). Examples of other lipid include phospholipid, sterols other than cholesterol, and PEG lipids other than the dimyristoylglycerol PEG represented by the formula (2).

The amount of other lipid in the lipid nanoparticle of the present invention is preferably 0 to 50 mol%, more preferably 0 to 30 mol%, further preferably 0 to 10 mol%, with respect to the total amount of lipids in the lipid nanoparticle. As used herein, when, for example, lipid nanoparticles contain an ionic lipid represented by the formula (1), cholesterol, dimyristoylglycerol PEG represented by the formula (2), and other lipid as constituent components, "the total amount of lipids in the lipid nanoparticle" means "the total amount of an ionic lipid represented by the formula (1), cholesterol, dimyristoylglycerol PEG represented by the formula (2), and other lipid". In the present specification, the "amount of B (mol%) with respect to A" means the "100 × amount of B (mol)/amount of A (mol)". For example, the "amount of other lipid (mol%) with respect to the total amount of lipids" means the "100 × amount of other lipid (mol)/total amount of lipid (mol)".

Most preferably, other lipids are not used in the present invention, i.e., lipids constituting the lipid nanoparticles of the present invention consist of an ionic lipid represented by the formula (1), cholesterol, and a dimyristoylglycerol PEG represented by the formula (2).

### Ionic lipid

The ionic lipid used in the present invention is an ionic lipid represented by the following formula (1) (sometimes to be abbreviated as "ionic lipid (1)" in the present specification). Only one kind of ionic lipid (1) may be used, or two or more kinds thereof may be used in combination. 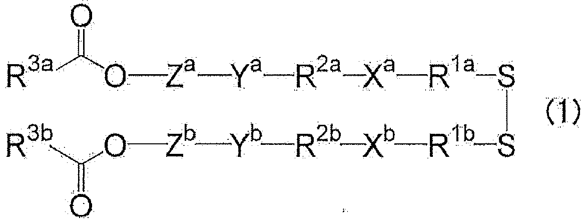 (in the formula (1),
R^{1a} and R^{1b} are each independently an alkylene group having 1 to 6 carbon atoms,
X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 to 16 carbon atoms and at least one aromatic ring, and optionally having a hetero atom, and
R^{3a} and R^{3b} are each independently a residue derived from a reaction product of a liposoluble vitamin having a hydroxyl group, and succinic anhydride or glutaric anhydride, or a residue derived from a reaction product of a sterol derivative having a hydroxyl group, and succinic anhydride or glutaric anhydride, or an aliphatic hydrocarbon group having 12 to 22 carbon atoms).

R^{1a} and R^{1b} are each independently an alkylene group having 1 to 6 carbon atoms, and may be linear or branched, preferably linear. The carbon number of the alkylene group is preferably 1 to 4, more preferably 1 to 2. Specific examples of the alkylene group having 1 to 6 carbon atoms include methylene group, ethylene group, trimethylene group, isopropylene group, tetramethylene group, isobutylene group, pentamethylene group, neopentylene group, and the like. Preferably, R^{1a} and R^{1b} are each independently a methylene group, an ethylene group, a trimethylene group, an isopropylene group, or a tetramethylene group, most preferably an ethylene group.

R^{1a} may be the same as or different from R^{1b}, and R^{1a} is preferably the same group as R^{1b}.

X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups, preferably each independently a cyclic alkylene tertiary amino group having 2 to 5 carbon atoms and 1 to 2 tertiary amino groups.

The alkyl group having 1 to 6 carbon atoms in the acyclic alkyl tertiary amino group having 1 to 6 carbon atoms and one tertiary amino group may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group, and the like, preferably methyl group, ethyl group, propyl group, or isopropyl group, most preferably methyl group.

A preferred specific structure of the acyclic alkyl tertiary amino group having 1 to 6 carbon atoms and one tertiary amino group is represented by X¹.

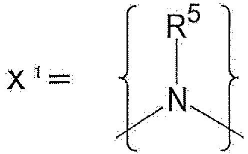

R⁵ in X¹ is an alkyl group having 1 to 6 carbon atoms, which may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl, 1,2-dimethylpropyl group, 2-methylbutyl group, 2-methylpentyl group, 3-methylpentyl group, 2,2-dimethylbutyl group, 2,3-dimethylbutyl group, cyclohexyl group, and the like, preferably methyl group, ethyl group, propyl group, or isopropyl group, most preferably methyl group.

The carbon number of the cyclic alkylene tertiary amino group having 2 to 5 carbon atoms and 1 to 2 tertiary amino groups is preferably 4 to 5. The cyclic alkylene tertiary amino group having 2 to 5 carbon atoms and 1 to 2 tertiary amino groups is specifically an aziridylene group, an azetidylene group, a pyrrolidylene group, a piperidylene group, an imidazolidylene group, or a piperazylene group, preferably a pyrrolidylene group, a piperidylene group, or a piperazylene group, most preferably a piperidylene group.

A preferred specific structure of the cyclic alkylene tertiary amino group having 2 to 5 carbon atoms and one tertiary amino group is represented by X².

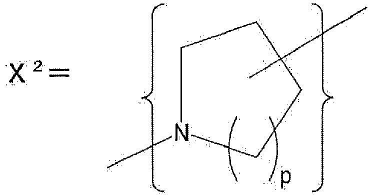

The p in X² is 1 or 2. When p is 1, X² is a pyrrolidylene group, and when p is 2, X² is a piperidylene group. Preferably, p is 2.

A preferred specific structure of the cyclic alkylene tertiary amino group having 2 to 5 carbon atoms and two tertiary amino groups is represented by X³.

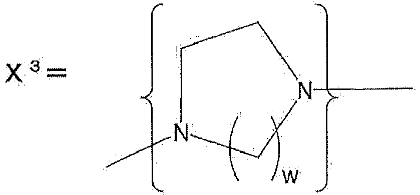

The w in X³ is 1 or 2. When w is 1, X³ is an imidazolidylene group, and when w is 2, X³ is a piperazylene group.

X^{a} may be the same as or different from X^{b}, and X^{a} is preferably the same group as X^{b}.

R^{2a} and R^{2b} are each independently an alkylene group or an oxydialkylene group each having not more than 8 carbon atoms, preferably each independently an alkylene group having not more than 8 carbon atoms.

The alkylene group having not more than 8 carbon atoms may be linear or branched, preferably linear. The number of carbons contained in the alkylene group is preferably not more than 6, most preferably not more than 4. Specific examples of the alkylene group having not more than 8 carbon atoms include methylene group, ethylene group, trimethylene group, isopropylene group, tetramethylene group, isobutylene group, pentamethylene group, hexamethylene group, heptamethylene group, octamethylene group, and the like, preferably methylene group, ethylene group, trimethylene group, or tetramethylene group, most preferably ethylene group.

In the present specification, the "oxydialkylene group having not more than 8 carbon atoms" means alkylene groups via an ether bond (alkylene-O-alkylene, in other words, "alkyleneoxyalkylene group"), wherein the total carbon number of the two alkylene groups present is 8 or below. The two alkylene groups present may be the same or different, preferably the same. Specific examples of the oxydialkylene group having not more than 8 carbon atoms include oxydimethylene group, oxydiethylene group, oxydi(trimethylene) group (i.e., trimethyleneoxytrimethylene group), oxydi(tetramethylene) group (i.e., tetramethyleneoxytetramethylene group), and the like. Preferably, it is an oxydimethylene group, an oxydiethylene group, or an oxydi(tetramethylene) group, most preferably an oxydiethylene group.

R^{2a} may be the same as or different from R^{2b}, and R^{2a} is preferably the same group as R^{2b}.

Y^{a} and Y^{b} are each independently an ester bond, an amide bond, a carbamate bond, an ether bond or a urea bond, preferably each independently an ester bond, an amide bond or a carbamate bond, more preferably each independently an ester bond or an amide bond, most preferably each an ester bond. The direction of the bond of Y^{a} and Y^{b} is not limited. When Y^{a} and Y^{b} are ester bonds, the structure of -Z^{a}-CO-O-R^{2a}- or -Z^{b}-CO-OR^{2b}- is preferably shown.

Y^{a} may be the same as or different from Y^{b}, and Y^{a} is preferably the same group as Y^{b}.

Z^{a} and Z^{b} are each independently a divalent group derived from an aromatic compound having 3 to 16 carbon atoms and at least one aromatic ring, and optionally having a hetero atom. The number of carbons contained in the aromatic compound is preferably 6 to 12, most preferably 6 to 7. The aromatic ring contained in the aromatic compound is preferably one.

As the kind of the aromatic ring contained in the aromatic compound having 3 to 16 carbon atoms, benzene ring, naphthalene ring, and anthracene ring can be mentioned for aromatic hydrocarbocycle, and imidazole ring, pyrazole ring, oxazole ring, isoxazole ring, thiazole ring, isothiazole ring, triazine ring, pyrrole ring, furan ring, thiophene ring, pyrimidine ring, pyridazine ring, pyrazine ring, pyridine ring, purine ring, pteridine ring, benzimidazole ring, indole ring, benzofuran ring, quinazoline ring, phthalazine ring, quinoline ring, isoquinoline ring, coumarin ring, chromone ring, benzodiazepine ring, phenoxazine ring, phenothiazine ring, acridine ring, and the like can be mentioned for aromatic heterocycle. It is preferably a benzene ring, a naphthalene ring, or an anthracene ring, most preferably a benzene ring.

The aromatic ring may have a substituent. Examples of the substituent include acyl group having 2 to 4 carbon atoms, alkoxycarbonyl group having 2 to 4 carbon atoms, alkylcarbamoyl group having 2 to 4 carbon atoms, acyloxy group having 2 to 4 carbon atoms, acylamino group having 2 to 4 carbon atoms, alkoxycarbonylamino group having 2 to 4 carbon atoms, fluorine atom, chlorine atom, bromine atom, iodine atom, alkylsulfanyl group having 1 to 4 carbon atoms, alkylsulfonyl group having 1 to 4 carbon atoms, arylsulfonyl group having 6 to 10 carbon atoms, nitro group, trifluoromethyl group, cyano group, alkyl group having 1 to 4 carbon atoms, ureido group, alkylureido group having 2 to 4 carbon atoms, alkoxy group having 1 to 4 carbon atoms, aryl group having 6 to 10 carbon atoms, aryloxy group having 6 to 10 carbon atoms, and the like. Preferred examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group, and the like.

A preferred specific structure of Z^{a} and Z^{b} is Z¹. 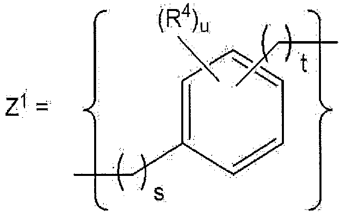 wherein s is an integer of 0 to 3, t is an integer of 0 to 3, u is an integer of 0 to 4, and R⁴ in the number of u are each independently a substituent.

The s in Z¹ is preferably an integer of 0 to 1, more preferably 0.

The t in Z¹ is preferably an integer of 0 to 2, more preferably 1.

The u in Z¹ is preferably an integer of 0 to 2, more preferably an integer of 0 to 1.

The R⁴ in Z¹ is a substituent of an aromatic ring (benzene ring) contained in the aromatic compound having 3 to 16 carbon atoms which does not inhibit the reaction in the synthesis process of an ionic lipid. Examples of the substituent include acyl group having 2 to 4 carbon atoms, alkoxycarbonyl group having 2 to 4 carbon atoms, alkylcarbamoyl group having 2 to 4 carbon atoms, acyloxy group having 2 to 4 carbon atoms, acylamino group having 2 to 4 carbon atoms, alkoxycarbonylamino group having 2 to 4 carbon atoms, fluorine atom, chlorine atom, bromine atom, iodine atom, alkylsulfanyl group having 1 to 4 carbon atoms, alkylsulfonyl group having 1 to 4 carbon atoms, arylsulfonyl group having 6 to 10 carbon atoms, nitro group, trifluoromethyl group, cyano group, alkyl group having 1 to 4 carbon atoms, ureido group, alkylureido group having 2 to 4 carbon atoms, alkoxy group having 1 to 4 carbon atoms, aryl group having 6 to 10 carbon atoms, aryloxy group having 6 to 10 carbon atoms, and the like. Preferred examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group, and the like. When R⁴ is present in plurality, each R⁴ may be the same or different.

Z^{a} may be the same as or different from Z^{b}, and Z^{a} is preferably the same group as Z^{b}.

R^{3a} and R^{3b} are each independently a residue derived from a reaction product of a liposoluble vitamin having a hydroxyl group, and succinic anhydride or glutaric anhydride, or a residue derived from a reaction product of a sterol derivative having a hydroxyl group, and succinic anhydride or glutaric anhydride, or an aliphatic hydrocarbon group having 12 to 22 carbon atoms, preferably each independently a residue derived from a reaction product of a liposoluble vitamin having a hydroxyl group, and succinic anhydride or glutaric anhydride, or an aliphatic hydrocarbon group having 12 to 22 carbon atoms, most preferably each independently an aliphatic hydrocarbon group having 12 to 22 carbon atoms.

Examples of the liposoluble vitamin having a hydroxyl group include retinol, ergosterol, 7-dehydrocholesterol, calciferol, cholecalciferol, dihydroergocalciferol, dihydrotachysterol, tocopherol, tocotrienol, and the like. The liposoluble vitamin having a hydroxyl group is preferably tocopherol.

Examples of the sterol derivative having a hydroxyl group include cholesterol, cholestanol, stigmasterol, β-sitosterol, lanosterol, ergosterol, and the like, preferably cholesterol or cholestanol.

The aliphatic hydrocarbon group having 12 to 22 carbon atoms may be linear or branched. The aliphatic hydrocarbon group may be saturated or unsaturated. In the case of an unsaturated aliphatic hydrocarbon group, the aliphatic hydrocarbon group generally contains 1 to 6, preferably 1 to 3, more preferably 1 to 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 13 to 19, most preferably 13 to 17. While the aliphatic hydrocarbon group includes an alkyl group, an alkenyl group, an alkynyl group, and the like, it is preferably an alkyl group or an alkenyl group. Specific examples of the aliphatic hydrocarbon group having 12 to 22 carbon atoms include dodecyl group, tridecyl group, tetradecyl group, pentadecyl group, hexadecyl group, heptadecyl group, octadecyl group, nonadecyl group, icosyl group, henicosyl group, docosyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, decadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, docosadienyl group, octadecatrienyl group, icosatrienyl group, icosatetraenyl group, icosapentaenyl group, docosahexaenyl group, isostearyl group, 1-hexylheptyl group, 1-hexylnonyl group, 1-octylnonyl group, 1-octylundecyl group, 1-decylundecyl group, and the like. The aliphatic hydrocarbon group having 12 to 22 carbon atoms is preferably a tridecyl group, a pentadecyl group, a heptadecyl group, a nonadecyl group, a heptadecenyl group, a heptadecadienyl group, or a 1-hexylnonyl group, particularly preferably a tridecyl group, a heptadecyl group, a heptadecenyl group, or a heptadecadienyl group.

In one embodiment of the present invention, the aliphatic hydrocarbon group having 12 to 22 carbon atoms for R^{3a} or R^{3b} is derived from fatty acid. In this case, the carbonyl carbon derived from fatty acid is contained in -CO-O- in the formula (1). A specific example of the aliphatic hydrocarbon group is a heptadecadienyl group when linoleic acid is used as the fatty acid, or a heptadecenyl group when oleic acid is used as the fatty acid.

R^{3a} may be the same as or different from R^{3b}, and R^{3a} is preferably the same group as R^{3b}.

In one embodiment of the present invention, R^{1a} is the same as R^{1b}, X^{a} is the same as X^{b}, R^{2a} is the same as R^{2b}, Y^{a} is the same as Y^{b}, Z^{a} is the same as Z^{b}, and R^{3a} is the same as R^{3b}.

Preferred examples of ionic lipid (1) include the following ionic lipids.

### [Ionic lipid (1-1)]

Ionic lipid (1) wherein
R^{1a} and R^{1b} are each independently an alkylene group having 1 to 6 carbon atoms (e.g., methylene group, ethylene group);
X^{a} and X^{b} are each independently an acyclic alkyl tertiary amino group having 1 to 6 carbon atoms and one tertiary amino group (e.g., -N(CH₃)-), or a cyclic alkylene tertiary amino group having 2 to 5 carbon atoms and 1 to 2 tertiary amino groups (e.g., piperidylene group);
R^{2a} and R^{2b} are each independently an alkylene group having not more than 8 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 to 16 carbon atoms and at least one aromatic ring, and optionally having a hetero atom (e.g., -C₆H₄-CH₂-, -CH₂-C₆H₄-CH₂-); and
R^{3a} and R^{3b} are each independently a residue derived from a reaction product of a liposoluble vitamin having a hydroxyl group (e.g., tocopherol), and succinic anhydride or glutaric anhydride, or an aliphatic hydrocarbon group having 12 to 22 carbon atoms (e.g., heptadecenyl group, heptadecadienyl group, 1-hexylnonyl group).

### [Ionic lipid (1-2)]

Ionic lipid (1) wherein
R^{1a} and R^{1b} are each independently an alkylene group having 1 to 4 carbon atoms (e.g., methylene group, ethylene group);
X^{a} and X^{b} are each independently an acyclic alkyl tertiary amino group having 1 to 3 carbon atoms and one tertiary amino group (e.g., -N(CH₃)-), or a cyclic alkylene tertiary amino group having 2 to 5 carbon atoms and one tertiary amino group (e.g., piperidylene group);
R^{2a} and R^{2b} are each independently an alkylene group having not more than 6 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 6 to 12 carbon atoms and one aromatic ring, and optionally having a hetero atom (e.g., - C₆H₄-CH₂-, -CH₂-C₆H₄-CH₂-) ; and
R^{3a} and R^{3b} are each independently a residue derived from a reaction product of a liposoluble vitamin having a hydroxyl group (e.g., tocopherol), and succinic anhydride, or an aliphatic hydrocarbon group having 13 to 19 carbon atoms (e.g., heptadecenyl group, heptadecadienyl group, 1-hexylnonyl group).

### [Ionic lipid (1-3)]

Ionic lipid (1) wherein
R^{1a} and R^{1b} are each independently an alkylene group having 1 to 2 carbon atoms (e.g., methylene group, ethylene group);
X^{a} and X^{b} are each independently X¹: 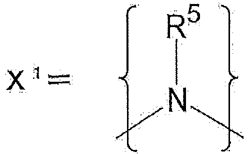
wherein R⁵ is an alkyl group having 1 to 3 carbon atoms (e.g., a methyl group), or X²: 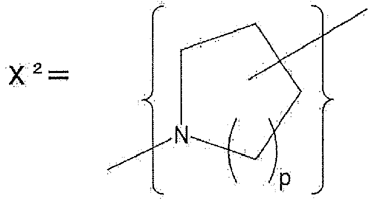 wherein p is 1 or 2;
R^{2a} and R^{2b} are each independently alkylene group having not more than 4 carbon atoms (e.g., methylene group, ethylene group, trimethylene group);
Y^{a} and Y^{b} are each independently an ester bond or an amide bond;
Z^{a} and Z^{b} are each independently Z¹: 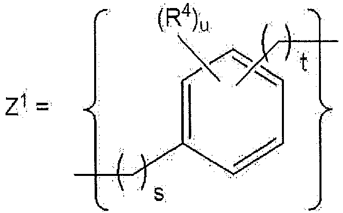
wherein s is an integer of 0 to 1, t is an integer of 0 to 2, u is an integer of 0 to 2 (preferably 0), and R⁴ in the number of u are each independently a substituent; and
R^{3a} and R^{3b} are each independently a residue derived from a reaction product of a liposoluble vitamin having a hydroxyl group (e.g., tocopherol) and succinic anhydride, or an aliphatic hydrocarbon group having 13 to 17 carbon atoms (e.g., heptadecenyl group, heptadecadienyl group, 1-hexylnonyl group).

Specific examples of ionic lipid (1) include the following O-Ph-P3C1, O-Ph-P4C1, O-Ph-P4C2, O-Bn-P4C2, E-Ph-P4C2, L-Ph-P4C2, HD-Ph-P4C2, O-Ph-amide-P4C2, and O-Ph-C3M.

**[Table 1-1]**

| name of ionic lipid | structure |
|---|---|
| O-Ph-P3C1 | 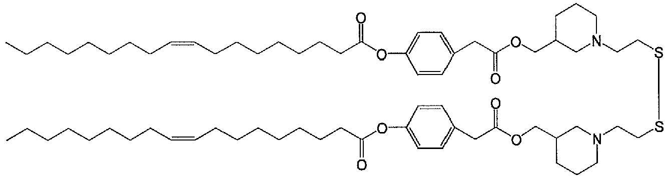 |
| O-Ph-P4C1 | 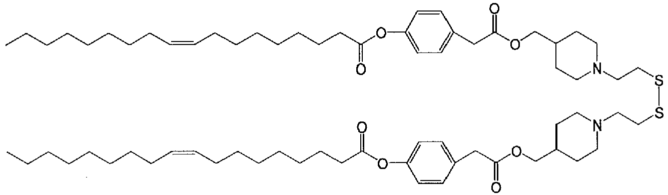 |
| O-Ph-P4C2 (or SS-OP) | 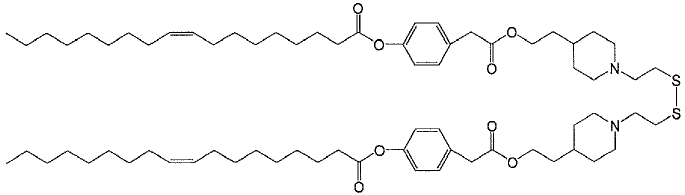 |
| O-Bn-P4C2 | 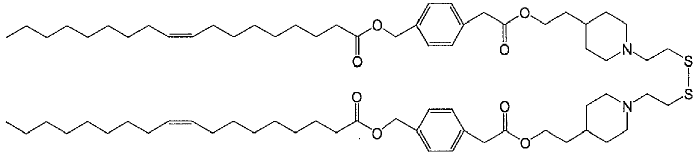 |
| E-Ph-P4C2 (or SS-EP) | 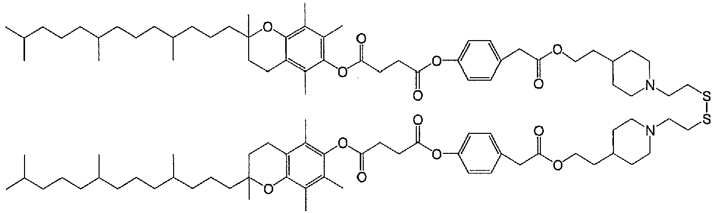 |

**[Table 1-2]**

| name of ionic lipid | structure |
|---|---|
| L-Ph-P4C2 | 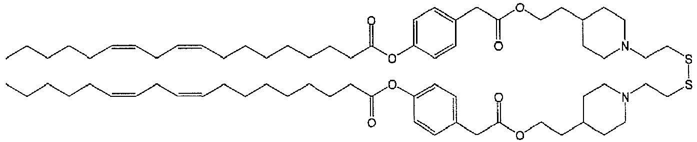 |
| HD-Ph-P4C2 | 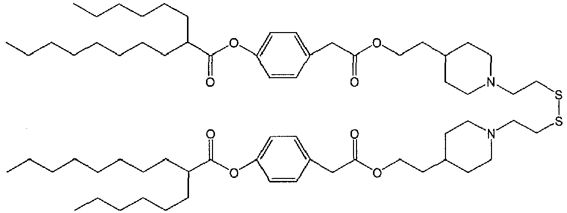 |
| O-Ph-amide-P4C2 | 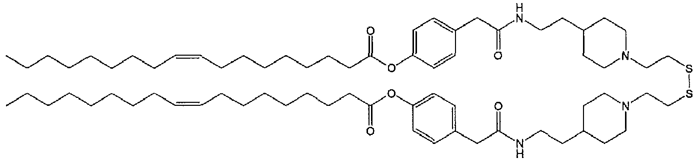 |
| O-Ph-C3M | 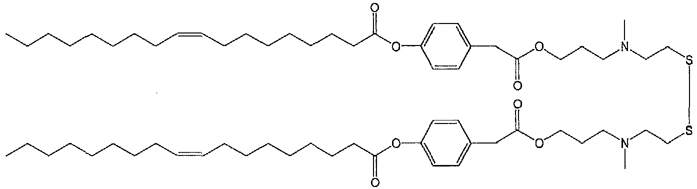 |

Among the specific examples of ionic lipid (1), SS-OP is preferred. That is, ionic lipid (1) is preferably an ionic lipid represented by the following formula.

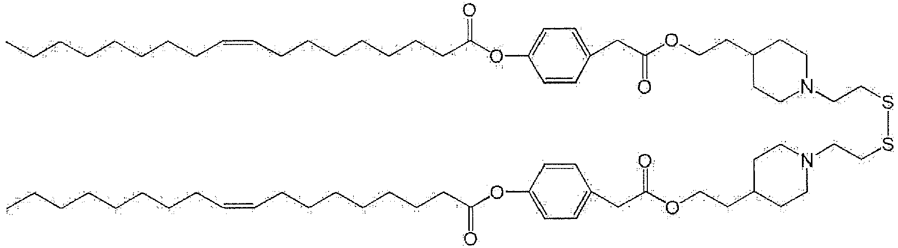

The amount of ionic lipid (1) in the lipid nanoparticle of the present invention is preferably 20 to 80 mol%, more preferably 30 to 70 mol%, further preferably 40 to 60 mol%, with respect to the total of ionic lipid (1) and cholesterol, from the aspects of efficiency of nucleic acid encapsulation, efficiency of intracellular release of nucleic acid, and stability of the lipid nanoparticles.

The ionic lipid (1) can be produced by a known method (e.g., the method described in WO 2019/188867 A1).

### Cholesterol

The lipid nanoparticles of the present invention contain cholesterol. The amount of the cholesterol in the lipid nanoparticle of the present invention is preferably 20 to 80 mol%, more preferably 30 to 70 mol%, further preferably 40 to 60 mol%, with respect to the total of ionic lipid (1) and cholesterol, from the aspects of efficiency of nucleic acid encapsulation, efficiency of intracellular release of nucleic acid, and stability of the lipid nanoparticles.

### Dimyristoylglycerol PEG

The lipid nanoparticles of the present invention contains a dimyristoylglycerol PEG represented by the formula (2):

CH₂(OR⁶)-CH(OR⁷)-CH₂(OR⁸) (2)

(in the formula (2), two of R⁶, R⁷, and R⁸ are myristoyl groups, and the remaining one is an alkyl group having 1 to 6 carbon atoms connected via a polyethylene glycol chain with a number average molecular weight of 1,000 to 3,000)
(sometimes to be abbreviated as "dimyristoylglycerol PEG (2)" in the present specification).

The number average molecular weight of the PEG chain in the formula (2) is 1,000 to 3,000, preferably 1,500 to 2,500. The number average molecular weight of PEG used to form the PEG chain can be measured by gel permeation chromatography (GPC).

The alkyl group having 1 to 6 carbon atoms may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group, and the like, preferably methyl group.

The amount of the dimyristoyl glycerol PEG (2) in the lipid nanoparticle of the present invention is preferably 3 to 15 mol%, more preferably 3 to 9 mol%, further preferably 3 to 6 mol%, with respect to the total of ionic lipid (1) and cholesterol, from the aspects of efficiency of nucleic acid encapsulation, efficiency of intracellular release of nucleic acid, and stability of the lipid nanoparticles.

### Phospholipid

The lipid nanoparticles of the present invention may contain phospholipids as lipids other than the ionic lipid represented by the formula (1), cholesterol, and the dimyristoylglycerol PEG represented by the formula (2). Only one kind of the phospholipid may be used, or two or more kinds thereof may be used in combination.

Examples of the phospholipid include 1,2-diacyl-sn-glycero-3-phosphocholine (PC), 1,2-diacyl-sn-glycero-3-phosphoethanolamine (PE), 1,2-diacyl-sn-glycero-3-phosphoserine (PS), 1,2-diacyl-sn-glycero-3-phosphoglycerol (PG), 1,2-diacyl-sn-glycero-3-phosphatidic acid (PA), lyso forms of these, and the like.

Specific examples of 1,2-diacyl-sn-glycero-3-phosphocholine (PC) include
1,2-didecanoyl-sn-glycero-3-phosphocholine (DDPC),
1,2-dilauroyl-sn-glycero-3-phosphocholine (DLPC),
1,2-dimyristoyl-sn-glycero-3-phosphocholine (DMPC),
1,2-dipalmitoyl-sn-glycero-3-phosphocholine (DPPC),
1,2-distearoyl-sn-glycero-3-phosphocholine (DSPC),
1,2-dioleoyl-sn-glycero-3-phosphocholine (DOPC),
1,2-dilinoleoyl-sn-glycero-3-phosphocholine (DLoPC),
1,2-dierucoyl-sn-glycero-3-phosphocholine (DEPC),
1-myristoyl-2-palmitoyl-sn-glycero-3-phosphocholine (MPPC),
1-myristoyl-2-stearoyl-sn-glycero-3-phosphocholine (MSPC),
1-palmitoyl-2-myristoyl-sn-glycero-3-phosphocholine (PMPC),
1-palmitoyl-2-stearoyl-sn-glycero-3-phosphocholine (PSPC),
1-palmitoyl-2-oleoyl-sn-glycero-3-phosphocholine (POPC), and
1-stearoyl-2-oleoyl-sn-glycero-3-phosphocholine (SOPC). In the present specification, phospholipid may be sometimes indicated with the abbreviation thereof. For example, 1,2-diacyl-sn-glycero-3-phosphocholine is sometimes indicated as PC, and 1,2-didecanoyl-sn-glycero-3-phosphocholine is sometimes indicated as DDPC.

Specific examples of 1,2-diacyl-sn-glycero-3-phosphoethanolamine (PE) include
1,2-didecanoyl-sn-glycero-3-phosphoethanolamine (DOPE),
1,2-dilauroyl-sn-glycero-3-phosphoethanolamine (DLPE),
1,2-dimyristoyl-sn-glycero-3-phosphoethanolamine (DMPE),
1,2-dipalmitoyl-sn-glycero-3-phosphoethanolamine (DPPE),
1,2-distearoyl-sn-glycero-3-phosphoethanolamine (DSPE),
1,2-dioleoyl-sn-glycero-3-phosphoethanolamine (DOPE),
1.2-dilinoleoyl-sn-glycero-3-phosphoethanolamine (DLoPE),
1,2-dierucoyl-sn-glycero-3-phosphoethanolamine (DEPE),
1-myristoyl-2-palmitoyl-sn-glycero-3-phosphoethanolamine (MPPE),
1-myristoyl-2-stearoyl-sn-glycero-3-phosphoethanolamine (MSPE),
1-palmitoyl-2-myristoyl-sn-glycero-3-phosphoethanolamine (PMPE),
1-palmitoyl-2-stearoyl-sn-glycero-3-phosphoethanolamine (PSPE),
1-palmitoyl-2-oleoyl-sn-glycero-3-phosphoethanolamine (POPE), and
1-stearoyl-2-oleoyl-sn-glycero-3-phosphoethanolamine (SOPE).

Specific examples of 1,2-diacyl-sn-glycero-3-phosphoserine (PS) include
1,2-didecanoyl-sn-glycero-3-phosphoserine (DDPS),
1,2-dilauroyl-sn-glycero-3-phosphoserine (DLPS),
1,2-dimyristoyl-sn-glycero-3-phosphoserine (DMPS),
1,2-dipalmitoyl-sn-glycero-3-phosphoserine (DPPS),
1,2-distearoyl-sn-glycero-3-phosphoserine (DSPS),
1,2-dioleoyl-sn-glycero-3-phosphoserine (DOPS),
1,2-dilinoleoyl-sn-glycero-3-phosphoserine (DLoPS),
1,2-dierucoyl-sn-glycero-3-phosphoserine (DEPS),
1-myristoyl-2-palmitoyl-sn-glycero-3-phosphoserine (MPPS),
1-myristoyl-2-stearoyl-sn-glycero-3-phosphoserine (MSPS),
1-palmitoyl-2-myristoyl-sn-glycero-3-phosphoserine (PMPS),
1-palmitoyl-2-stearoyl-sn-glycero-3-phosphoserine (PSPS),
1-palmitoyl-2-oleoyl-sn-glycero-3-phosphoserine (POPS), and
1-stearoyl-2-oleoyl-sn-glycero-3-phosphoserine (SOPS).

Specific examples of 1,2-diacyl-sn-glycero-3-phosphoglycerol (PG) include
1,2-didecanoyl-sn-glycero-3-phosphoglycerol (DDPG),
1,2-dilauroyl-sn-glycero-3-phosphoglycerol (DLPG),
1,2-dimyristoyl-sn-glycero-3-phosphoglycerol (DMPG),
1,2-dipalmitoyl-sn-glycero-3-phosphoglycerol (DPPG),
1,2-distearoyl-sn-glycero-3-phosphoglycerol (DSPG),
1,2-dioleoyl-sn-glycero-3-phosphoglycerol (DOPG),
1,2-dilinoleoyl-sn-glycero-3-phosphoglycerol (DLoPG),
1,2-dierucoyl-sn-glycero-3-phosphoglycerol (DEPG),
1-myristoyl-2-palmitoyl-sn-glycero-3-phosphoglycerol (MPPG),
1-myristoyl-2-stearoyl-sn-glycero-3-phosphoglycerol (MSPG),
1-palmitoyl-2-myristoyl-sn-glycero-3-phosphoglycerol (PMPG),
1-palmitoyl-2-stearoyl-sn-glycero-3-phosphoglycerol (PSPG),
1-palmitoyl-2-oleoyl-sn-glycero-3-phosphoglycerol (POPG), and
1-stearoyl-2-oleoyl-sn-glycero-3-phosphoglycerol (SOPG).

Specific examples of 1,2-diacyl-sn-glycero-3-phosphatidic acid (PA) include
1,2-didecanoyl-sn-glycero-3-phosphatidic acid (DOPA),
1,2-dilauroyl-sn-glycero-3-phosphatidic acid (DLPA),
1,2-dimyristoyl-sn-glycero-3-phosphatidic acid (DMPA),
1,2-dipalmitoyl-sn-glycero-3-phosphatidic acid (DPPA),
1,2-distearoyl-sn-glycero-3-phosphatidic acid (DSPA),
1,2-dioleoyl-sn-glycero-3-phosphatidic acid (DOPA),
1,2-dilinoleoyl-sn-glycero-3-phosphatidic acid (DLoPA),
1,2-dierucoyl-sn-glycero-3-phosphatidic acid (DEPA),
1-myristoyl-2-palmitoyl-sn-glycero-3-phosphatidic acid (MPPA),
1-myristoyl-2-stearoyl-sn-glycero-3-phosphatidic acid (MSPA),
1-palmitoyl-2-myristoyl-sn-glycero-3-phosphatidic acid (PMPA),
1-palmitoyl-2-stearoyl-sn-glycero-3-phosphatidic acid (PSPA),
1-palmitoyl-2-oleoyl-sn-glycero-3-phosphatidic acid (POPA), and
1-stearoyl-2-oleoyl-sn-glycero-3-phosphatidic acid (SOPA).

Phospholipid is preferably PC and/or PE, more preferably at least one selected from the group consisting of DOPC, POPC, DOPE, and POPE.

When phospholipid is used, the amount thereof in the lipid nanoparticle of the present invention is preferably 5 to 25 mol%, more preferably 5 to 20 mol%, further preferably 10 to 20 mol%, with respect to the total of ionic lipid (1) and cholesterol, from the aspects of efficiency of nucleic acid encapsulation, efficiency of intracellular release of nucleic acid, and stability of the lipid nanoparticles.

### Production method of lipid nanoparticles

The lipid nanoparticles of the present invention can be produced by dispersing a lipid material containing ionic lipid (1), cholesterol, and dimyristoylglycerol PEG (2) in a suitable dispersion medium (for example, aqueous dispersion medium, alcoholic dispersion medium), and performing an operation to induce organization as necessary.

Examples of the "operation to induce organization" for producing the lipid nanoparticles of the present invention include methods known per se such as ethanol dilution method using a microchannel or vortex, simple hydration method, sonication, heating, vortex, ether injecting method, French press method, cholic acid method, Ca²⁺ fusion method, freeze-thaw method, reversed-phase evaporation method, and the like, preferably ethanol dilution method using a microchannel or vortex, further preferably ethanol dilution method using a microchannel. In the ethanol dilution method using a microchannel, for example, a dispersion containing lipid nanoparticles can be produced by mixing an acidic buffer containing a nucleic acid and an ethanol solution of a lipid by using NanoAssemblr (Precision NanoSystems). The dispersion produced by this method contains lipid nanoparticles and a dispersion medium (acidic buffer and ethanol). The dispersion medium (particularly ethanol) can be removed, the dispersion medium (particularly buffer) can be exchanged, and the like by operations such as ultrafiltration, dialysis, dilution, and the like.

### Method for delivering nucleic acid to lymphatic endothelial cell

The present invention also provides a method for delivering a nucleic acid to a lymphatic endothelial cell, including administering the lipid nanoparticles encapsulating the nucleic acid of the present invention to a subject. The aforementioned lipid nanoparticles are preferably administered subcutaneously to a subject.

Examples of the nucleic acid include, but are not limited to, DNA, RNA, chimera nucleic acid of RNA, DNA/RNA hybrid and the like. While any single-stranded to triple-stranded nucleic acid can be used, it is preferably single-stranded or double-stranded. The nucleic acid may be a nucleotide having N-glycoside of purine or pyrimidine base, an oligomer having a non-nucleotide backbone (e.g., commercially available peptide nucleic acid (PNA) etc.), or an oligomer containing a special bond (said oligomer containing a nucleotide having a configuration permitting base pairing or attachment of base, which are found in DNA and RNA) and the like.

Furthermore, the nucleic acid may be, for example, a nucleic acid added with known modification, a nucleic acid with a label known in the field, a nucleic acid with a cap, a methylated nucleic acid, a nucleic acid with one or more natural nucleotides substituted by an analog, a nucleic acid with modified nucleotide, a nucleic acid having a non-charge bond (e.g., methylphosphonate, phosphotriester, phosphoramidate, carbamate and the like), a nucleic acid having a charged bond or sulfur-containing bond (e.g., phosphorothioate, phosphorodithioate and the like), a nucleic acid having a side chain group such as protein (e.g., nuclease, nuclease inhibitor, toxin, antibody, signal peptide, poly-L-lysine, and the like), sugar (e.g., monosaccharide and the like), and the like, a nucleic acid containing an intercalating compound (e.g., acridine, psoralen, and the like), a nucleic acid containing a chelate compound (e.g., metal, radioactive metal, boron, oxidative metal, and the like), a nucleic acid containing an alkylating agent, or a nucleic acid containing a modified bond (e.g., α anomer-type nucleic acid and the like).

The type of the DNA that can be used in the present invention is not particularly limited, and can be selected as appropriate according to the purpose of use. Examples of the DNA include plasmid DNA, cDNA, antisense DNA, chromosomal DNA, PAC, BAC, CpG oligosaccharide, and the like. Preferred are plasmid DNA, cDNA and antisense DNA, and more preferred is plasmid DNA. A circular DNA such as plasmid DNA and the like can be digested as appropriate with a restriction enzyme and the like, and also used as a linear DNA.

The type of the RNA that can be used in the present invention is not particularly limited, and can be selected as appropriate according to the purpose of use. Examples of the RNA include siRNA, miRNA, shRNA, antisense RNA, messenger RNA (mRNA), single-stranded RNA genome, double-stranded RNA genome, RNA replicon, transfer RNA, ribosomal RNA, and the like, with preference given to siRNA, miRNA, shRNA, mRNA, antisense RNA, and RNA replicon.

The nucleic acid used in the present invention is preferably purified by a method generally used by those of ordinary skill in the art.

The nucleic acid to be used in the present invention is preferably one having a preventive and/or therapeutic activity against a given disease (prophylactic/therapeutic nucleic acid). Examples of such nucleic acid include nucleic acids used for so-called gene therapy, and the like.

The particle size of the lipid nanoparticle encapsulating a nucleic acid is not particularly limited, and is preferably 10 nm to 500 nm, more preferably 30 nm to 300 nm. The particle size can be measured by using a particle size distribution measuring device such as Zetasizer Nano (Malvern) or the like. The particle size of the lipid nanoparticle encapsulating a nucleic acid can be appropriately adjusted by the production method thereof.

The surface charge (zeta potential) of the lipid nanoparticles encapsulating a nucleic acid is not particularly limited and preferably -15 to +15 mV, more preferably -10 to +10 mV. In conventional transgene, particles with positively charged surfaces have been mainly used. This is useful as a method for promoting electrostatic interactions with heparin sulfate on the negatively-charged cell surface to enhance uptake into cells. However, the positive surface potential may suppress (a) nucleic acid release from the carrier due to the interaction with a nucleic acid to be delivered in the cell, or (b) protein synthesis due to the interaction between mRNA and a nucleic acid to be delivered. This problem can be solved by adjusting the surface potential (zeta potential) to fall within the above-mentioned range. The surface potential (zeta potential) can be measured using a zeta potential measuring apparatus such as Zetasizer Nano or the like. The surface potential (zeta potential) of the lipid nanoparticles can be adjusted by the composition of the constituent components of the lipid nanoparticles.

By administering the lipid nanoparticles encapsulating nucleic acid of the present invention to a subject, the lipid nanoparticles reach and contact lymphatic endothelial cells, and the nucleic acid encapsulated in the lipid nanoparticle is delivered to the lymphatic endothelial cells in vivo. The subject to which the lipid nanoparticles can be administered is not particularly limited and, for example, mammals (e.g., human, monkey, mouse, rat, hamster, bovine, etc.), birds (e.g., chicken, ostrich, etc.), amphibia (e.g., frog etc.), fishes (e.g., zebrafish, medaka (Oryzias latipes), etc.), and the like can be mentioned. The subject of administration of the lipid nanoparticles is preferably human or other mammal.

The administration method of the lipid nanoparticles encapsulating nucleic acid to a subject is not particularly limited as long as the lipid nanoparticles can deliver nucleic acid to lymphatic endothelial cells, and an administration method known per se (e.g., oral administration, parenteral administration (e.g., transnasal administration, intravenous administration, intramuscular administration, topical administration, transdermal administration, subcutaneous administration, intraperitoneal administration, spray, etc.), etc.) can be appropriately selected. As the administration method, subcutaneous administration is preferred. The dose of the lipid nanoparticles can be appropriately selected in consideration of the kind of the subject of administration, administration method, and the like.

The lipid nanoparticles of the present invention may be used as they are, or mixed with a pharmaceutically acceptable carrier and can be produced as an oral agent (e.g., tablet, capsule agent, etc.) or a parenteral agent (e.g., transdermal preparation, injection, etc.), preferably a parenteral agent (more preferably, subcutaneous preparation).

As the pharmaceutically acceptable carrier, those conventionally used as formulation materials are used. For example, in solid preparations, excipient, lubricant, binder, disintegrant, and the like are used, and in liquid preparations, solvent, solubilizing agent, suspending agent, isotonicity agent, buffering agent, soothing agent, and the like are used. Where necessary, formulation additives such as antiseptic, antioxidant, colorant, sweetening agent, and the like can also be used.

For example, in the case of subcutaneous preparations, they are used in the form of subcutaneous injection or subcutaneous transfusion.

### [Example]

The contents of the present invention are further explained in detail in the following by using Examples and the like, but the present invention is not limited in any way by the Examples and the like.

In the following Examples and the like, ionic lipid (1) is shown by the name listed in the aforementioned Table. The abbreviations used in the following Examples and the like each mean the following.
Chol: cholesterol
DiD: 1,1'-dioctadecyl-3,3,3',3'-tetramethylindodicarbocyanine perchlorate
DMG-PEG2000: 1,2-dimyristoyl-rac-glycerol, methoxypolyethylene glycol (PEG number average molecular weight (Mn): 2000)
DSG-PEG2000: 1,2-distearoyl-rac-glycerol, methoxypolyethylene glycol (PEG number average molecular weight (Mn): 2000)
PBS: phosphate buffered saline

### [Production Example 1] Preparation of siRNA-encapsulating LNP

### (1) Preparation of ethanol solution of lipid

For the preparation of an ethanol solution of lipid, 10 mM SS-OP and 10 mM Chol were mixed at a ratio of 1:1 so that the total lipid amount was 1800 nmol, and 3 mM DMG-PEG2000 ethanol solution or 1 mM DSG-PEG2000 ethanol solution was added to the desired proportions. Ethanol was added to make the total amount 500 µL.

### (2) Preparation of acidic buffer solution of nucleic acid

The acidic buffer solution of nucleic acid was prepared by weighing out a siRNA solution against 2 µg/µL vascular endothelial growth factor receptor 3 (VEGFR3) so that the amount of siRNA was 14.4 µg, and adding 20 mM acidic malate buffer containing 30 mM NaCl (pH 3.0) to a total amount of 1200 µL.

### (3) LNP preparation by ethanol dilution method

Using the microfluidic device Nanoassemblr (registered trademark; Nepagene), 1200 µL of an acidic buffer solution of nucleic acid and 500 µL of an ethanol solution of lipid were mixed at flow rates of 12 mL/min and 4 mL/min, respectively, and 1 mL of LNP solution was obtained. 1 mL of the LNP solution was transferred to Amicon Ultra 4 (Nihon Millipore K.K.) and 3.5 mL of PBS was added. After centrifugation at 25°C, 1000 g, 5 min, inversion blending was thoroughly performed, and the mixture was centrifuged again under the same conditions, and concentrated by ultrafiltration to about 1.5 mL. The volume was made up to 4.5 mL using PBS and concentrated again to about 1.5 mL. Finally, the solution was diluted to a lipid concentration of 0.5 mM using PBS. Particles with a small particle size were obtained by using ultrapure water as a solvent for ultrafiltration instead of PBS.

### [Experimental Example 1] Measurement of particle size and surface potential of various siRNA-encapsulating LNPs

The particle diameter and surface potential of the six types of siRNA-encapsulating LNPs prepared in Production Example 1 with different kinds and composition ratios of PEG lipids were measured by a dynamic light scattering method (Zetasizer Nano; Malvern). The results are shown in Table 2.

**[Table 2]**

| | particle size (zeta average) | particle size (number average) | PdI | zeta potential |
|---|---|---|---|---|
| Example 1 DMG-PEG2000 3 mol% | 71±3 nm | 50±0 nm | 0.10±0.01 | -3.8±0.2 mv |
| Example 2 DMG-PEG2000 6 mol% | 63±9 nm | 39±8 nm | 0.21±0.07 | -5.5±1.1 mv |
| Comparative Example 1 DMG-PEG2000 1 mol% | 133±9 nm | 91±5 nm | 0.1610.03 | -4.3±0.7 mv |
| Comparative Example 2 DSG-PEG2000 1 mol% | 175±12 nm | 120±14 nm | 0.20±0.02 | -2.610.7 mv |
| Comparative Example 3 DSG-PEG2000 3 mol% | 71±6 nm | 4612 nm | 0.16±0.03 | -2.3±0.6 mv |
| Comparative Example 4 DSG-PEG2000 6 mol% | 60±8 nm | 36±2 nm | 0.22±0.08 | -0.6+0.2 mv |

| | | | | |
|---|---|---|---|---|
| (In Table, mol% shows molar ratio with respect to total amount of SS-OP and Chol.) | | | | |

### [Experimental Example 2] Evaluation of in vivo LNP uptake

### (1) Administration to mouse tail and collection of skin

Six types of siRNA-encapsulating LNPs (Examples 1 and 2 and Comparative Examples 1 to 4) obtained in Production Example 1 were fluorescently labeled with DiD, and 20 µL was subcutaneously administered to 6-week-old female C57/BL6J mice (10 nmol as lipid amount was administered). It was administered with the needle heading toward the tip of the tail from the midpoint of the tail in a supine position. Eighteen hours after administration, mice were euthanized and the tails thereof were collected. The tail was excised in a length of about 1.5 cm centered at the administration site. The skin was removed from the bone using a razor and tweezers.

### (2) Obtainment of cell suspension from mouse subcutaneous tissue

For the digestive enzyme solution for mouse skin tissue, 10 mg of collagenage IV (Worthington), 10 µL, of 120 mM calcium chloride solution, and 10 µL of 4 mg/mL DNAase (Worthington) were weighed per mouse, and D-MEM (Nacalai Tesque) (pH 7.4, containing 2% FBS, 10 mM HEPES) was added to a total amount of 1 mL. 1 mL of the digestive enzyme solution was transferred per well of a 12-well plate, and the skin was immersed therein with the dermis side down. It was incubated under the conditions of 37°C, 500 rpm, 30 min. After peeling off the epidermis, a metal mesh was placed under the skin and a spatula was used to scrape the cells from the dermis. 1 mL of autoclaved PBS solution containing 0.5% v/w bovine serum albumin and 0.1% v/w sodium azide (as FACS buffer) was added, and the total volume was transferred to a 5 mL tube through a 70 µm) cell strainer (Hitech). It was centrifuged under the conditions of 4°C, 500 g, 5 min, and the supernatant was discarded. The pellet was suspended in 1 mL of FACS buffer, and the entire amount was transferred to a 1.5 mL tube through a nylon mesh (SANSYO). It was centrifuged under the same conditions and the supernatant was discarded.

### (3) Antibody staining

The pellet was suspended in FACS buffer (50 µL), antimouse CD16/32 antibody (Biolegend) (1 µL) was added, and the mixture was incubated for 10 min. Brilliant Violet 605-anti mouse CD45 antibody (Biolegend) (1 µL), Brilliant Violet 421-anti mouse podoplanin antibody (Biolegend) (1.25 µL), and PE-anti mouse CD31 antibody (Biolegend) (5 µL) were added, and the mixture was incubated for 25 min. FACS buffer was added to a total amount of 500 µL, 7AAD (Biolegend) (5 µL) was added, and the mixture was incubated for 5 min. It was centrifuged under the conditions of 4°C, 500 g, 5 min, and the supernatant was discarded. The pellet was suspended in FACS buffer (500 µL), and the entire amount was transferred through a nylon mesh to a tube for flow cytometer analysis.

### (4) Flow cytometry analysis

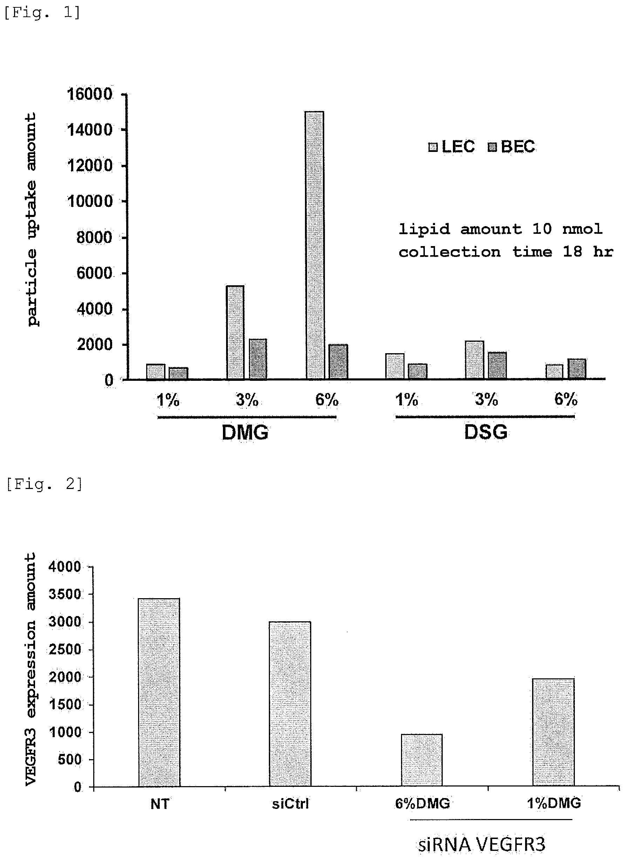

Novocyte; Agilent Technologies (formerly ACEA Biosciences) was used as a flow cytometer. Live cells were gated by 7AAD and, using CD45-negative, podoplanin-positive, and CD31-positive cell population as lymphatic endothelial cells, and CD45-negative, podoplanin-negative, and CD31-positive cell population as blood vessel endothelial cells, analysis was performed until the number of lymphatic endothelial cells reached about 1500 cells. The geometric mean of DiD fluorescence of each cell population was used as an index of uptake. The results are shown in Fig. 1. In Fig. 1, DMG or DSG indicates the results using DMG-PEG2000 or DSG-PEG2000, respectively. By using not less than 3% of DMG-PEG2000, the amount and selectivity of uptake into lymphatic endothelial cells increased.

### [Experimental Example 3] Evaluation of in vivo gene knockdown activity

### (1) Administration to mouse tail and collection of skin tissue

The siRNA-encapsulating LNPs (Example 2 and Comparative Example 1) prepared in Production Example 1 (20 µL) were subcutaneously administered to 6-week-old female C57/BL6J mice (0.1 µg as siRNA was administered). As a control, non-specific siRNA (0.1 µg) was subcutaneous administered to a mouse (siCtrl). It was administered with the needle heading toward the tip of the tail from the midpoint of the tail in a supine position. Forty-eight hours after administration, mice were euthanized and the tails thereof were collected. The tail was excised in a length of about 1.5 cm centered at the administration site. The skin was removed from the bone using a razor and tweezers.

### (2) Obtainment of cell suspension from mouse subcutaneous tissue

Cell suspensions were obtained from subcutaneous tissue of mouse by the method described in [Experimental Example 2].

### (3) Antibody staining

The pellet was suspended in FACS buffer (10 µL), antimouse CD16/32 antibody (Biolegend) (1 µL) was added, and the mixture was incubated for 10 min. Brilliant Violet 605-anti mouse CD45 antibody (Biolegend) (1 µL), Brilliant Violet 421-anti mouse podoplanin antibody (Biolegend) (1.25 µL), PE-anti mouse CD31 antibody (Biolegend) (5 µL), and APC-anti mouse VEGFR3 antibody (R&D) (10 µL) were added, and the mixture was incubated for 120 min. FACS buffer was added to a total amount of 500 µL, 7AAD (Biolegend) (5 µL) was added, and the mixture was incubated for 5 min. It was centrifuged under the conditions of 4°C, 500 g, 5 min, and the supernatant was discarded. The pellet was suspended in FACS buffer (500 µL), and the entire amount was transferred through a nylon mesh to a tube for flow cytometer analysis.

### (4) Flow cytometry analysis

Novocyte; Agilent Technologies (formerly ACEA Biosciences) was used as a flow cytometer. Live cells were gated by 7AAD and, using CD45-negative, podoplanin-positive, and CD31-positive cell population as lymphatic endothelial cells, and CD45-negative, podoplanin-negative, and CD31-positive cell population as blood vessel endothelial cells, analysis was performed until the number of lymphatic endothelial cells reached about 1500 cells. The VEGFR3 gene expression of each LNP was compared based on the geometric mean of the fluorescence intensity of APC in the lymphatic endothelial cell fraction of the LNP non-administration group (NT). The results are shown in Fig. 2. In Fig. 2, NT, siCtrl, and DMG respectively indicate the results of the non-administration group, the results of the non-specific siRNA administration group, and the results using DMG-PEG2000. LNP using 6 mol% DMG-PEG2000 (Example 2) significantly suppressed VEGFR3 expression as compared with LNP using 1 mol% DMG-PEG2000 (Comparative Example 1).

### [Industrial Applicability]

The LNP of the present invention can selectively deliver drugs such as nucleic acids encapsulated in particles to lymphatic tissues, and are therefore useful as a drug delivery system (DDS) to lymphatic tissues.

This application is based on a patent application No. 2021-161239 filed in Japan, the contents of which are incorporated in full herein.

## Claims

1. A lipid nanoparticle used for delivering a nucleic acid to a lymphatic endothelial cell, comprising
an ionic lipid represented by the formula (1): 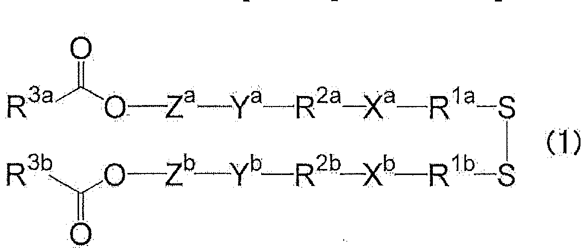 (in the formula (1),
R^{1a} and R^{1b} are each independently an alkylene group having 1 to 6 carbon atoms,
X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 to 16 carbon atoms and at least one aromatic ring, and optionally having a hetero atom, and
R^{3a} and R^{3b} are each independently a residue derived from a reaction product of a liposoluble vitamin having a hydroxyl group, and succinic anhydride or glutaric anhydride, or a residue derived from a reaction product of a sterol derivative having a hydroxyl group, and succinic anhydride or glutaric anhydride, or an aliphatic hydrocarbon group having 12 to 22 carbon atoms),
cholesterol, and
a dimyristoylglycerol PEG represented by the formula (2):
CH₂(OR⁵)-CH(OR⁷)-CH₂(OR⁸) (2)
(in the formula (2),
two of R⁶, R⁷, and R⁸ are myristoyl groups, and the remaining one is an alkyl group having 1 to 6 carbon atoms connected via a polyethylene glycol chain with a number average molecular weight of 1,000 to 3,000),
wherein an amount of the dimyristoylglycerol PEG represented by the formula (2) is not less than 3 mol% with respect to the total of the ionic lipid represented by the formula (1) and the cholesterol.

2. The lipid nanoparticle according to claim 1, wherein lipids constituting the lipid nanoparticle consist of the ionic lipid represented by the formula (1), the cholesterol, and the dimyristoylglycerol PEG represented by the formula (2).

3. The lipid nanoparticle according to claim 1 or 2, wherein the ionic lipid represented by the formula (1) is an ionic lipid represented by the following formula:

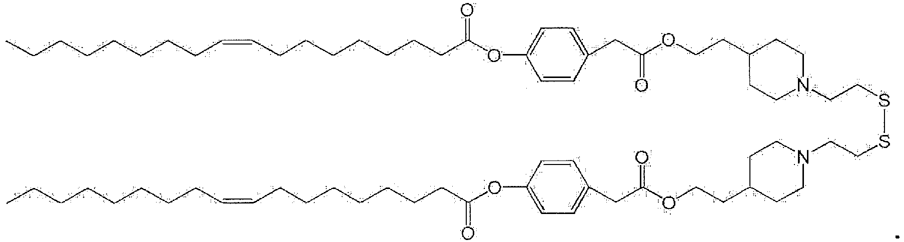

4. A method for delivering a nucleic acid to a lymphatic endothelial cell, comprising subcutaneously administering the lipid nanoparticle according to any one of claims 1 to 3 that encapsulates the nucleic acid to a subject.
